# EUROPÄISCHE PATENTSCHRIFT

(11) **EP 2 297 126 B1**
(45) Veröffentlichungstag und Bekanntmachung des Hinweises auf die Patenterteilung: **14.12.2011**
(21) Anmeldenummer: 09765774.6
(22) Anmeldetag: 10.06.2009
(51) Int. Cl.: C07D 303/16

(54) **VERFAHREN ZUR HERSTELLUNG VON GLYCIDYLESTERN**
METHOD FOR PRODUCING GLYCIDYL ESTERS
PROCÉDÉ DE PRÉPARATION D'ESTERS DE GLYCIDYLE

(30) Priorität: 18.06.2008 EP 08158482
(43) Veröffentlichungstag der Anmeldung: 23.03.2011
(73) Patentinhaber: BASF SE, 67056 Ludwigshafen (DE)
(72) Erfinder: GUMLICH, Kai, 68159 Mannheim (DE); TELES, Joaquim Henrique, 67166 Otterstadt (DE); MERTEN, Roland, 69469 Weinheim (DE)
(86) Internationale Anmeldenummer: PCT/EP2009/057134
(87) Internationale Veröffentlichungsnummer: WO 2009/153194

(56) Entgegenhaltungen:
- EP-A- 0 212 409
- DE-C- 845 937
- FR-A- 1 100 845
- US-A- 4 111 965
- US-A- 4 257 966
- US-A- 4 276 223

## Beschreibung

Die Erfindung betrifft ein Verfahren zur Herstellung von Glycidylestern, welches dadurch gekennzeichnet, dass Carbonatester der Formel I 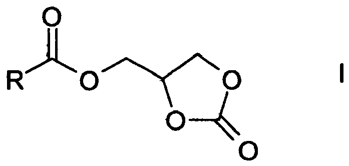 in Gegenwart eines homogenen Katalysators unter Abspaltung von Kohlendioxid zu Glycidylestern der Formel II 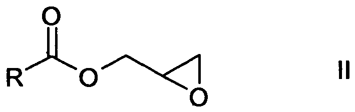 umgesetzt werden, wobei R in den obigen Formeln für einen organischen Rest mit 1 bis 20 C-Atomen steht.

Carbonatester der Formel I benötigen Glycerin als Ausgangsstoff, die Bedeutung derartiger Carbonatester wächst daher mit der Verfügbarkeit von Glycerin.

Glycidylverbindungen können aus entsprechenden Carbonatverbindungen unter Abspaltung von Kohlendioxid erhalten werden. In WO 98/40371 ist ein derartiges Verfahren beschrieben; von der angegebenen Formel für geeignete Carbonatverbindungen werden auch Ester umfasst (R = Acyl). Als Katalysator wird in WO 98/40371 ein Zeolith, d.h. ein heterogener Katalysator verwendet.

Aus EP-A 582 201 und US 2856413 ist bekannt, dass Carbonatverbindungen in Gegenwart von Metallsalzen als Katalysatoren bei hohen Temperaturen (125 bis 275 °C) in die entsprechenden Glycidylverbindungen, z.B. das Glycerincarbonat in Glycidol, überführt werden können.

Bei derartigen Verfahren ist grundsätzlich eine gute Ausbeute und hohe Selektivität erwünscht.

Das Verfahren soll außerdem leicht durchzuführen-sein und die Verfahrensprodukte sollen leicht abgetrennt und von Nebenprodukten, und/oder Katalysatoren gereinigt werden können. Die Katalysatoren sollen ohne großen Aufwand wieder verwendbar sein.

Aufgabe der Erfindung war, ein entsprechendes Verfahren zur Verfügung zu stellen.

Demgemäß wurde das eingangs definierte Verfahren gefunden.

Bei dem erfindungsgemäßen Verfahren werden Carbonatester der Formel I umgesetzt.

Der Rest R in Formel I steht für einen organischen Rest mit 1 bis 20 C-Atomen, insbesondere 1 bis 15 C-Atomen. Der organische Rest kann auch Hetereoatome wie z. B. Sauerstoff, Stickstoff und Schwefel enthalten.

Vorzugsweise steht R für einen Kohlenwasserstoffrest mit 1 bis 20 C-Atomen, insbesondere 1 bis 1 bis 15 C-Atomen.

Besonders bevorzugt handelt es sich um einen aliphatischen Kohlenwasserstoffrest, insbesondere um eine Alkylgruppe oder Alkenylgruppe

Als ganz besonders bevorzugte Reste R seien eine C1 bis C15, insbesondere C6 bis C12 Alkylgruppe und eine C3 bis C10 Alkenylgruppe genannt, diese Gruppen können linear oder verzweigt sein.

Insbesondere handelt es sich um die folgenden Carbonatester der Formel III und IV:
Formel III: Carbonatester der 2-Ethylhexansäure 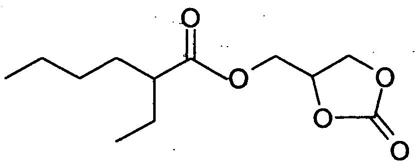
Formel IV: Carbonatester der Methacrylsäure

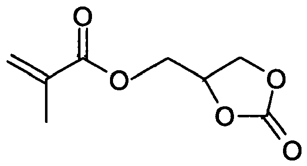

Weiterhin sind Carbonatester der 2-Propylheptansäure, der Neodecansäure und der Isononansäure von besonderer Bedeutung.

Das erfindungsgemäße Verfahren wird in Gegenwart eines homogenen Katalysators durchgeführt. Unter einem homogenen Katalysator wird hier ein Katalysator verstanden, der sich zumindest teilweise in den Edukten oder einem mitverwendeten Lösemittel unter den Reaktionsbedingungen löst.

Vorzugsweise sollte der homogene Katalysator in dem Edukt der Formel I oder dem mit verwendeten Lösemittel eine Löslichkeit von mindestens 5 Gew.- teilen, besonders bevorzugt von mindestens 20 Gew. teilen und ganz besonders bevorzugt von mindestens 50 Gew. teilen in 100 Gew.- teilen Edukt oder Lösemittel bei 20 °C, 1 bar haben.

Insbesondere handelt es sich bei dem homogenen Katalysator um ein Salz. Das Salz kann aus organischen oder anorganischen Anionen oder Kationen bestehen.

Besonders bevorzugt handelt es sich bei dem Katalysator um eine ionische Flüssigkeit. Unter dem Begriff "ionische Flüssigkeit wird ein Salz verstanden, welches bei Temperaturen kleiner 100°C, insbesondere bei Temperaturen kleiner 50°C und besonders bevorzugt bereits bei Raumtemperatur (21°C) flüssig ist. Alle Angaben sind dabei auf Normaldruck (1 bar) bezogen).

Als ionische Flüssigkeit bevorzugt ist ein Salz mit einem organischen Kation. Das organische Kation ist insbesondere ein heterocyclisches Ringsystem mit mindestens einem, vorzugsweise zwei Stickstoffatomen als Teil des Ringsystems.

In Betracht kommt insbesondere ein Pyridinium- oder ein Imidazolium-kation.

Ganz besonders bevorzugt handelt es sich bei dem Katalysator um ein Imidazoliumsalz.

In Betracht kommen insbesondere Imidazoliumsalze der Formel V 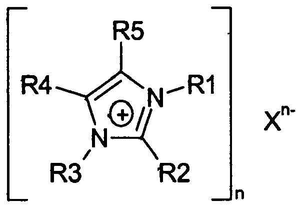 worin
R1 und R3 jeweils unabhängig voneinander für einen organischen Rest mit 1 bis 20 C-Atomen stehen
R2, R4, und R5 unabhängig voneinander für ein H-Atom oder für einen organischen Rest mit 1 bis 20 C-Atomen stehen
X für ein Anion
n für 1, 2 oder 3 steht.

R1 und R3 stehen vorzugsweise unabhängig voneinander für einen organischen Rest mit 1 bis 10 C-Atomen. Der organische Rest kann auch noch weitere Heteroatome, insbesondere Sauerstoffatome, zum Beispiel Hydroxylgruppen, Ethergruppen, Estergruppen oder Carbonylgruppen enthalten.

Insbesondere stehen R1 und R3 für einen Kohlenwasserstoffrest, der außer Kohlenstoff und Wasserstoff allenfalls noch Hydroxylgruppen, Ethergruppen, Estergruppen oder Carbonylgruppen enthalten kann.

R1 und R3 stehen besonders bevorzugt unabhängig voneinander für einen Kohlenwasserstoffrest mit 1 bis 20 C- Atomen, insbesondere mit 1 bis 10 C-Atomen, der keine sonstigen Heteroatome, z. B. Sauerstoff oder Stickstoff, enthält. Der Kohlenwasserstoffrest kann aliphatisch (wobei auch ungesättigte aliphatische Gruppen eingeschlossen sind) oder aromatisch sein oder sowohl aromatische als auch aliphatische Gruppen enthalten. Vorzugsweise stehen R1 und R2 für einen aliphatischen Kohlenwasserstoffrest.

Als Kohlenwasserstoffreste genannt seien z.B. die Phenylgruppe, Benzylgruppe, eine durch eine oder mehrere C1 bis C4 Alkylgruppen substituierte Phenylgruppe oder Benzylgruppe, Alkylgruppen und Alkenylgruppen, insbesondere die Allylgruppe.

Ganz besonders bevorzugt stehen R1 und R3 für eine C1 bis C10 Alkylgruppe. Als Alkylgruppe ist eine C1 bis C6 Alkylgruppe besonders bevorzugt, in einer besonderen Ausführungsform handelt es sich bei der Alkylgruppe um eine C1 bis C4 Alkylgruppe.

Ganz besonders bevorzugt stehen R1 und R3 unabhängig voneinander für eine Methyl-, Ethyl-, n-Propyl-, Isopropyl-, n-Butyl-, sec.Butyl oder tert.Butylgruppe, eine, wobei die Methyl-, Ethyl-, n-Propyl- und n-Butylgruppe besondere Bedeutung haben.

In einer besonderen Ausführungsform stehen
R1 und R3 für eine Methylgruppe,
R1 und R3 für eine Ethylgruppe,
R1 für eine Methylgruppe und R3 für eine Ethylgruppe
R1 für eine Methylgruppe und R3 für eine n Propylgruppe
R1 für eine Methylgruppe und R3 für eine n Butylgruppe

R2, R4, und R5 stehen unabhängig voneinander für ein H-Atom oder für einen organischen Rest mit 1 bis 20 C-Atomen stehen, wobei R4 und R5 auch zusammen einen aliphatischen oder aromatischen Ring ausbilden können. Der organische Rest kann neben Kohlenstoff und Wasserstoff auch Heteroatome wie Stickstoff oder Sauerstoff enthalten; vorzugsweise kann er Sauerstoff enthalten, insbesondere in Form von Hydroxylgruppen, Estergruppen, Ethergruppen oder Carbonylgruppen.

Insbesondere stehen R2, R4 und R5 unabhängig voneinander für ein H-Atom oder einen Kohlenwasserstoffrest, der außer Kohlenstoff und Wasserstoff allenfalls noch Hydroxylgruppen, Ethergruppen, Estergruppen oder Carbonylgruppen enthalten kann.

R2, R4 und R5 stehen bevorzugt unabhängig voneinander für ein Wasserstoffatom oder einen Kohlenwasserstoffrest mit 1 bis 20 C- Atomen, insbesondere mit 1 bis 10 C-Atomen, der keine sonstigen Heteroatome, z. B. Sauerstoff oder Stickstoff, enthält. Der Kohlenwasserstoffrest kann aliphatisch (wobei auch ungesättigte aliphatische Gruppen eingeschlossen sind) oder aromatisch sein oder sowohl aus aromatischen als auch aus aliphatischen Gruppen bestehen, wobei R4 und R5 auch einen aromatischen oder aliphatischen Kohlewasserstoffring ausbilden können, der gegebenenfalls durch weitere aromatische oder aliphatische Kohlenwasserstoffgruppen substituiert sein kann (die Anzahl der C-Atome des gegebenenfalls substituierten Kohlenwasserstoffrings einschließlich der Substituenten kann dabei vorzugsweise maximal 40, insbesondere maximal 20, besonders bevorzugt maximal 15 bzw. maximal 10 betragen).
Als Kohlenwasserstoffreste genannt seien z.B. die Phenylgruppe, eine Benzylgruppe, eine durch eine oder mehrere C1 bis C4 Alkylgruppen substituierte Phenylgruppe oder Benzylgruppe, Alkylgruppen, Alkenylgruppen und, im Falle, dass R4 und R5 einen Ring ausbilden, ein durch R4 und R5 ausgebildeter aromatischer 5 - oder 6 Ring, ein Cyclohexen - oder Cyclopenten, wobei diese Ringsysteme insbesondere durch eine oder mehrere C1 bis C10, insbesondere C1 bis C4 Alkylgruppen substituiert sein können.
Als Kohlenwasserstoffrest sind aliphatische Kohlenwasserstoffreste bevorzugt.

Besonders bevorzugt stehen R2, R4 und R5 unabhängig voneinander für ein H-Atom, eine C1 bis C8 Alkylgruppe oder eine C1-C8 Alkenylgruppe, z. B. eine Allygruppe,

Ganz besonders bevorzugt stehen R2, R4 und R5 unabhängig voneinander für ein H-Atom, eine Methyl-, Ethyl-, n Propyl-, Isopropyl-, n Butyl-, sec.Butyl oder tert.Butylgruppe, wobei die Methyl-, Ethyl-, n Propyl- und n Butylgruppe besondere Bedeutung haben.

In einer besonderen Ausführungsform stehen R2, R4 und R5 für ein H-Atom oder-eine C1 bis C4 Alkylgruppe, insbesondere stehen R2, R4 und R5 für ein H-Atom.

Als Einzelfälle für die Kationen der Verbindungen der Formel I seien genannt:
1-Butyl-3-methyl-imidazolium (R1= Butyl, R3 = Methyl)
1-Butyl-3-ethyl-imidazolium (R1=Butyl, R3=Ethyl)
1,3-Di-methyl-imidazolium (R1= Methyl, R3 =Methyl)
1-Ethyl-3-methyl-imidazolium (R1=Ethyl, R3 =Methyl)
1-Ethyl-2,3 dimethyl- imidazolium (R1 =Ethyl, R2=Methyl, R3=Methyl)

In Formel V steht n für 1, 2 oder 3; das Anion hat entsprechend ein, zwei oder drei negative Ladungen und entsprechend liegen im Salz ein, zwei oder drei Imidazoliumkationen vor.

Bevorzugt steht n für 1 oder 2, besonders bevorzugt steht n für 1; das Anion ist daher besonders bevorzugt einwertig.

In Formel V steht X für ein Anion. Als Anionen sind organische und anorganische Anionen geeignet, die zusammen mit dem Kation eine ionische Flüssigkeit ergeben. Als Anionen genannt seien insbesondere Halogenide, sogenannte Pseudohalogenide wie CN-, SCN-, OCN-, Sulfate, Phosphate, Anionen mit organischen Gruppen, insbesondere Alkylester, z.B. Phosphonate oder Sulfonate

Als Anion wird im Rahmen dieser Erfindung vorzugsweise kein Chlorid verwendet, da die Gegenwart von Chlor oder Chloriden für einige Verwendungen der Verbindungen unerwünscht ist.

Der homogene Katalysator enthält daher vorzugsweise kein von Chlor oder Chlorid. Es handelt sich vorzugsweise um einen Chlor -freien Katalysator.

Als Anion besonders bevorzugt ist generell ein Jodid. Bei dem homogenen Katalysator handelt es sich daher vorzugsweise um ein Jodid.

Besonders bevorzugt ist ein Imidazoliumjodid, insbesondere ein Imidazoliumjodid der obigen Imidazolium-kationen.

Die Menge des homogenen Katalysators beträgt vorzugsweise 0,1 bis 50 Gewichtsteile (Gew.-teile), besonders bevorzugt 0,5 bis 40 Gewichtsteile auf 100 Gew.-teile Edukt der Formel I. Insbesondere wird der homogene Katalysator in Mengen von mindestens 0,1, insbesondere 0,5 Gew. teilen eingesetzt; die Menge ist im Allgemeinen nicht größer als 20 Gew. Teile, bzw. insbesondere nicht größer als 10 Gew. teile, bezogen auf 100 Gew.-teile Edukt der Formel I.

Bei dem Verfahren kann zusätzliches Lösemittel, wenn gewünscht, verwendet werden. Die Zugabe von Lösemittel kann insbesondere aus verfahrenstechnischen-Gründen, z. B. zur Wärmeaufnahme hilfreich sein.
Statt im Edukt kann der homogene Katalysator, wenn gewünscht, auch im Lösemittel gelöst werden. Im Falle der ionischen Flüssigkeit als Katalysator kann die ionische Flüssigkeit die Aufgaben eines Lösemittels mit übernehmen. Wird die ionische Flüssigkeit nur in geringen Mengen eingesetzt, kann auch hier gegebenenfalls die Mitverwendung von Lösemittel vorteilhaft sein.

Als Lösemittel in Betracht kommen solche, die unter den Reaktionsbedingungen flüssig sind und nicht mit der entstandenen Epoxygruppe (Glycidylring) reagieren. Das sind z. B. Polyether, deren endständige Hydroxygruppen mit Alkylgruppen verethert sind, derartige Polyether sind z. B. unter der Bezeichnung Plurafac® im Handel erhältlich. Das Lösemittel ist vorzugsweise zumindest in begrenzten mengen mit dem Edukt mischbar, besonders bevorzugt ist es in beliebigen Mengen mit dem Edukt mischbar.

Das Verfahren kann diskontinuierlich, halbkontinuierlich oder kontinuierlich durchgeführt werden.

Die Temperaturen betragen vorzugsweise 100 bis 275°C, insbesondere 120 bis 200°C.

Die Umsetzung wird vorzugsweise bei vermindertem Druck durchgeführt, das der erhaltene Glycidylester vorzugsweise direkt bei vermindertem Druck aus der Reaktionsmischung abdestilliert wird.

Die erhaltenen Produkte weisen statt des Carbonatrings einen Glycidylring auf.

Ausgehend vom Carbonat der Formel III erhält man den Glycidylester der Formel VI :

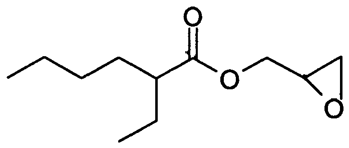

Ausgehend vom Carbonat der Formel IV erhält man den Glycidylester der formel VII :

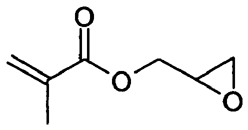

Die Glycidylester können nach dem erfindungsgemäßen Verfahren in einfacher Weise hergestellt werden. Es werden hohe Ausbeuten und Selektivitäten erreicht.

Besondere Bedeutung hat das erfindungsgemäße verfahren auch für Glycerin als Rohstoff.
Glycerinmonoester sind in einfacher Weise durch Umsetzung von Glycerin mit Carbonsäuren erhältlich. Die Glycerinmonoester können durch Umsetzung mit CO₂ oder Dialkylcarbonaten, wie Dimethylcarbonat oder Diethylcarbonat, in die Verbindungen der Formel I überführt werden und mit dem erfindungsgemäßen verfahren nun in einfacher Weise zu den Glycidylestern der Formel II umgesetzt werden.

### Beispiele

Neben dem 2-Ethylhexansäure Glycerincarbonat Ester (2-EHGCE) wurde auch der entsprechende Methacrylsäureester (MAAGCE) als Edukt verwendet.

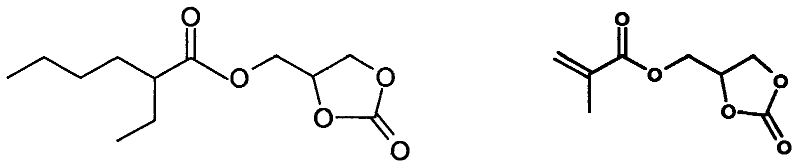

Folgende Katalysatoren kamen zum Einsatz:

| | | |
|---|---|---|
| Nal | CaF | Butylmethylimidazolium iodid (BMIM lodid) |
| Kl | Na₂SO₄ | |
| Li₂CO₃ | Li₃PO₄ | |

Die Versuche wurden in zwei Varianten einer durchgeführt:
   a) Batch-Versuche: Sowohl das Carbonat als auch der Katalysator wurden im Sumpfkolben einer Destillationsapparatur vorgelegt. Anschließend wurde das Gemisch im Vakuum erhitzt und der Glycidylester destillierte über.
   b) Semi-batch Versuche: Für diese Versuche wurde lediglich der Katalysator in einem inerten Lösungsmittel im Sumpfkolben vorgelegt (im Falle der lLs wurde zum Teil auf das Lösungsmittel verzichtet). Das Carbonat wurde kontinuierlich zugetropft sobald der Sumpfkolben die erforderlichen Drücke und Temperaturen erreicht worden waren.

### Batch-versuche

Beispiel Nr. 1) 2-Ethylhexansäureglycidylester durch CO₂ Abspaltung mit Nal als Katalysator (Vergleichsversuch, NaJ wird im Edukt supendiert)

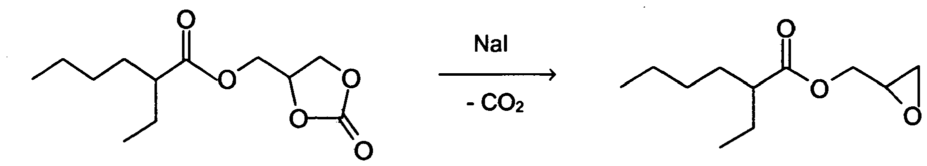

### Apparatur

| | | |
|---|---|---|
| 250 ml-Vierhalskolben | Magnetrührer | Kapillare für N₂-Einperlung |
| Thermometer | 25 cm Vigreux-Kolonne | |
| Tropftrichter | Destillationsbrücke + Spinne | |

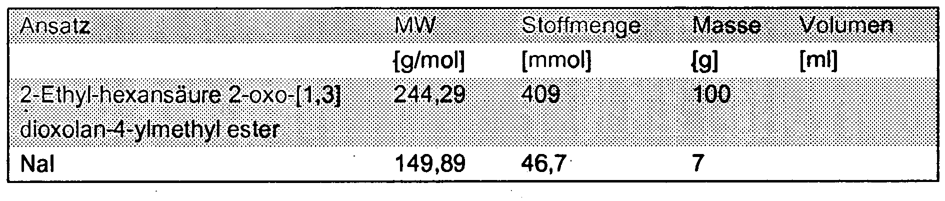

| Prozeßschritt | |
|---|---|
| • | Carbonatester mit Nal vorlegen |
| • | N₂-Einperlung über Kapillare in Betrieb nehmen |
| • | Auf 100 mbar evakuieren |
| • | Auf 180°C erhitzen |
| • | Druck auf 11 mbar reduzieren |
| • | Bei einer Sumpftemperatur von ca. 180-189°C, und einer Kopftemperatur von 110-114°C destilliert das Produkt über. |

Ausbeute: 79 %

| | | |
|---|---|---|
| Epoxidäquivalente: | | 222 g/eq |
| Wassergehalt: | | <0,01 g/100g |
| Farbzahl: | 36 APHA | |

### Semi-Batch Versuche

### Beispiel 11) 2-Ethylhexansäureglycidylester durch CO₂ Abspaltung mit BMIM lodid als Katalysator in Plurafac LF 431

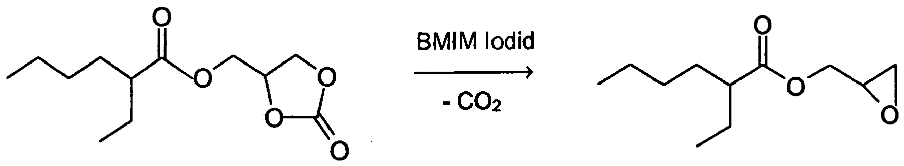

### Apparatur

| | | |
|---|---|---|
| 250 ml-Vierhalskolben | | Kapillare für N₂- |
| | Magnetrührer | Einperlung |
| Thermometer | 25 cm Vigreux-Kolonne | |
| Tropftrichter | Destillationsbrücke + Spinne | |

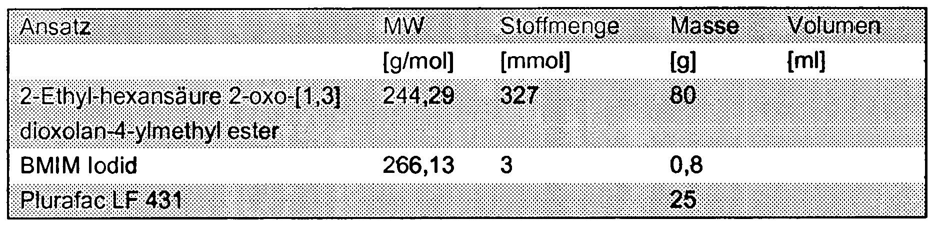

| Prozeßschritt | |
|---|---|
| • | Carbonatester mit BMIM lodid in Plurafac LF431 vorlegen |
| • | Auf 1 mbar evakuieren |
| • | Auf 155°C erhitzen |
| • | Ester innerhalb von 45 h zutropfen |
| • | Bei einer Sumpftemperatur von ca. 154-200°C und einer Kopftemperatur von 97-98°C destilliert das Produkt über. |

Ausbeute: 82 %

**Tab. 1 Versuchsübersicht**

| Versuch Nr | Edukt | Menge | Katalysator | Menge Edukt | LM bzgl. | Ausbeute |
|---|---|---|---|---|---|---|
| | | [g] | | [Gew%] | | [%] |
| 1 | 2-EHGCE | 100 | Nal | 7 | - | 79 |
| 2 | 2-EHGCE | 71,6 | Na₂SO₄ | 7 | - | 0,3 |
| 3 | MAAGCE | 135,7 | KI | 7 | - | 2 |
| 4 | 2-EHGCE | 51,2 | Li₂CO₃ | 7 | - | 0 |
| 5 | 2-EHGCE | 40,7 | CaF | 7 | - | 0 |
| 6 | 2-EHGCE | 36,2 | KI | 7 | - | 23 |
| 7 | 2-EHGCE | 90 | Li₃PO₄ | 7 | - | 5,6 |
| | | | | | | |
| 8 | 2-EHGCE | 73,8 | BMIM Iodid | 11,3 | - | 98 |
| | | | | | | |
| 9 | 2-EHGCE | 80 | BMIM Iodid | 10 | - | 90 |
| 10 | 2-EHGCE | 80 | BMIM Iodid | 31,3 | - | 81 |
| 11 | 2-EHGCE | 80 | BMIM Iodid | 1 | Plurafac LF 431 | 82 |
| | | | | | | |
| 12 | MAAGCE | 80 | BMIM lodid | 31,3 | - | 8,6 |
| 13 | MAAGCE | 80 | BMIM lodid | 31,3 | - | 3,5 |
| 14 | 2-EHGCE | 80 | BMIM lodid | 7 | - | 79 |

Versuch 10, 11, 12 und 13 wurden in semi-batch - Fahrweise durchgeführt. Die anderen Versuche wurden in batch- Fahrweise durchgeführt.

Versuche 1 bis 7 sind Vergleichsversuche, das Salz wurde im Edukt suspendiert.

## Patentansprüche

1. Verfahren zur Herstellung von Glycidylestern, **dadurch gekennzeichnet, dass** Carbonatester der Formel I 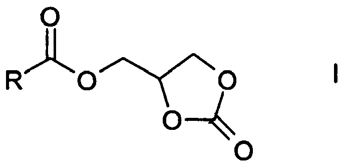 in Gegenwart eines homogenen Katalysators unter Abspaltung von Kohlendioxid zu Glycidylestern der Formel II 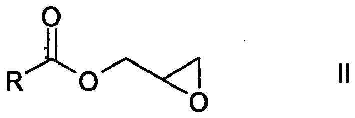 umgesetzt werden, wobei R in den obigen Formeln für einen organischen Rest mit 1 bis 20 C-Atomen steht.

2. Verfahren gemäß Anspruch 1, **dadurch gekennzeichnet, dass** R für einen Kohlenwasserstoffrest steht

3. Verfahren gemäß Anspruch 1 oder 2, **dadurch gekennzeichnet, dass** R für eine C1 bis C20 Alkylgruppe steht

4. Verfahren gemäß einem der Ansprüche 1 bis 3, **dadurch gekennzeichnet, dass** der homogene Katalysator in dem Edukt der Formel I oder in einem verwendeten Lösemittel eine Löslichkeit von mindestens 5 Gew.- teilen in 100 Gew.-teilen Edukt oder Lösemittel bei 20 °C, 1 bar hat

5. Verfahren gemäß einem der Ansprüche 1 bis 4, **dadurch gekennzeichnet, dass** es sich bei dem homogenen Katalysator um ein bei Temperaturen kleiner 100 °C, (1 bar), flüssiges Salz (ionische Flüssigkeit) handelt

6. Verfahren gemäß Anspruch 5, **dadurch gekennzeichnet, dass** es sich bei der ionischen Flüssigkeit um ein Imidazoliumsalz handelt

7. Verfahren gemäß einem der Ansprüche 1 bis 6, **dadurch gekennzeichnet, dass** es sich um einen Chlor-freien Katalysator handelt

8. Verfahren gemäß einem der Ansprüche 1 bis 7, **dadurch gekennzeichnet, dass** es sich bei dem homogenen Katalysator um ein Jodid handelt

9. Verfahren gemäß einem der Ansprüche 1 bis 8, **dadurch gekennzeichnet, dass** es sich bei dem homogenen Katalysator um ein Imidazoliumjodid handelt

10. Verfahren gemäß einem der Ansprüche 1 bis 9, **dadurch gekennzeichnet, dass** die Menge des Katalysators 1 bis 50 Gewichtsteile (Gew.-teile) auf 100 Gew.-teile Edukt der Formel I beträgt.

11. Verfahren gemäß einem der Ansprüche 1 bis 10, **dadurch gekennzeichnet, dass** zunächst aus einer Carbonsäure durch Umsetzung mit Glycerin ein Glycerinmonoester hergestellt wird, dieser Glycerinmonoester in den Carbonatester der Formel überführt wird.

## Claims

1. A process for preparing glycidyl esters, wherein carbonate esters of the formula I 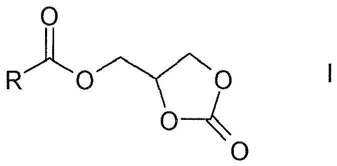 are reacted in the presence of a homogeneous catalyst with elimination of carbon dioxide to form glycidyl esters of the formula II 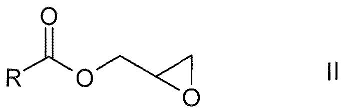 where R in the above formulae is an organic radical having from 1 to 20 carbon atoms.

2. The process according to claim 1, wherein R is a hydrocarbon radical.

3. The process according to claim 1 or 2, wherein R is a C1-C20-alkyl group.

4. The process according to any of claims 1 to 3, wherein the homogeneous catalyst has a solubility in the starting material of the formula I or in a solvent used of at least 5 parts by weight in 100 parts by weight of starting material or solvent at 20°C, 1 bar.

5. The process according to any of claims 1 to 4, wherein the homogeneous catalyst is a salt which is liquid at a temperature of less than 100°C (1 bar) (ionic liquid).

6. The process according to claim 5, wherein the ionic liquid is an imidazolium salt.

7. The process according to any of claims 1 to 6, wherein the catalyst is a chlorine-free catalyst.

8. The process according to any of claims 1 to 7, wherein the homogeneous catalyst is an iodide.

9. The process according to any of claims 1 to 8, wherein the homogeneous catalyst is an imidazolium iodide.

10. The process according to any of claims 1 to 9, wherein the amount of catalyst is from 1 to 50 parts by weight per 100 parts by weight of starting material of the formula I.

11. The process according to any of claims 1 to 10, wherein a glyceryl monoester is firstly prepared from a carboxylic acid by reaction with glycerol and this glyceryl monoester is converted into the carbonate ester of the formula I.

## Revendications

1. Procédé pour la préparation d'esters de glycidyle, **caractérisé en ce qu'**on transforme des esters de carbonate de formule I 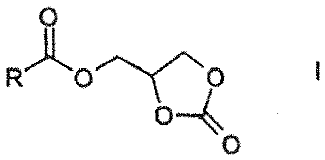 en présence d'un catalyseur homogène avec dissociation de dioxyde de carbone en esters de glycidyle de formule II 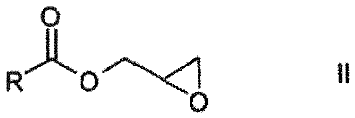 où R, dans les formules ci-dessus, représente un radical organique comprenant 1 à 20 atomes de carbone.

2. Procédé selon la revendication 1, **caractérisé en ce que** R représente un radical hydrocarboné.

3. Procédé selon la revendication 1 ou 2, **caractérisé en ce que** R représente un groupe C₁-C₂₀-alkyle.

4. Procédé selon l'une quelconque des revendications 1 à 3, **caractérisé en ce que** le catalyseur homogène dans le produit de départ de formule I ou dans un solvant utilisé présente une solubilité d'au moins 5 parties en poids dans 100 parties en poids de produit de départ ou de solvant à 20°C, 1 bar.

5. Procédé selon l'une quelconque des revendications 1 à 4, **caractérisé en ce qu'**il s'agit, pour le catalyseur homogène, d'un sel liquide (liquide ionique) à des températures inférieures à 100°C (1 bar).

6. Procédé selon la revendication 5, **caractérisé en ce qu'**il s'agit, pour le liquide ionique, d'un sel d'imidazolium.

7. Procédé selon l'une quelconque des revendications 1 à 6, **caractérisé en ce qu'**il s'agit d'un catalyseur exempt de chlore.

8. Procédé selon l'une quelconque des revendications 1 à 7, **caractérisé en ce qu'**il s'agit, pour le catalyseur homogène, d'un iodure.

9. Procédé selon l'une quelconque des revendications 1 à 8, **caractérisé en ce qu'**il s`agit, pour le catalyseur homogène, d'un iodure d'imidazolium.

10. Procédé selon l'une quelconque des revendications 1 à 9, **caractérisé en ce que** la quantité de catalyseur est de 1 à 50 parties en poids pour 100 parties en poids de produit de départ de formule I.

11. Procédé selon l'une quelconque des revendications 1 à 10, **caractérisé en ce qu'**on prépare d'abord, à partir d'un acide carboxylique, par transformation avec du glycérol, un monoester de glycérol, puis on transforme ce monoester de glycérol en ester de carbonate de formule I.
